(19) Europäisches Patentamt
European Patent Office
Office européen des brevets

(11) EP 1 267 820 B1

(12) **EUROPÄISCHE PATENTSCHRIFT**

(45) Veröffentlichungstag und Bekanntmachung des Hinweises auf die Patenterteilung:
**02.11.2005 Patentblatt 2005/44**

(21) Anmeldenummer: **01929461.0**

(22) Anmeldetag: **27.03.2001**

(51) Int Cl.[7]: **A61K 7/42**, C11D 3/42, D06M 13/402, D06M 15/59, D06M 13/224

(86) Internationale Anmeldenummer:
**PCT/EP2001/003470**

(87) Internationale Veröffentlichungsnummer:
**WO 2001/072935 (04.10.2001 Gazette 2001/40)**

(54) **TEXTILFASERAFFINE UV-ABSORBER-MISCHUNG**

UV-ABSORBING MIXTURE WITH TEXTILE FIBRE AFFINITY

MELANGE ABSORBANT LES UV, A AFFINITE POUR LES FIBRES TEXTILES

(84) Benannte Vertragsstaaten:
**AT BE CH CY DE DK ES FI FR GB GR IE IT LI LU MC NL PT SE TR**

(30) Priorität: **28.03.2000 DE 10015086**

(43) Veröffentlichungstag der Anmeldung:
**02.01.2003 Patentblatt 2003/01**

(73) Patentinhaber: **BASF Aktiengesellschaft**
**67056 Ludwigshafen (DE)**

(72) Erfinder:
- **HEIDENFELDER, Thomas**
**67354 Römerberg (DE)**
- **WAGENBLAST, Gerhard**
**67157 Wachenheim (DE)**
- **DETERING, Jürgen**
**67117 Limburgerhof (DE)**
- **HABECK, Thorsten**
**67149 Meckenheim (DE)**

(56) Entgegenhaltungen:

| | |
|---|---|
| **EP-A- 0 659 877** | **WO-A-97/44422** |
| **WO-A-99/06014** | **DE-A- 19 643 515** |
| **DE-A- 19 918 967** | **FR-A- 2 658 075** |
| **US-A- 5 770 183** | **US-A- 6 033 649** |

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 267 820 B1

## Beschreibung

Die vorliegende Erfindung betrifft die Verwendung einer Mischung aus

| | | |
|---|---|---|
| (A) | 10 bis 90 Gew.-% | mindestens eines $C_6$- bis $C_{18}$-Alkylesters oder $C_5$- bis $C_8$-Cycloalkylesters der 2-Cyano-3,3-diphenylacrylsäure und |
| (B) | 90 bis 10 Gew.-% | mindestens einer weiteren Verbindung, welche mindestens ein UV-Absorptionsmaximum im Bereich von 280 bis 450 nm aufweist und ausgewählt ist aus der Gruppe der<br>(a) Phenylbenzotriazole,<br>(b) Dibenzoylmethane,<br>(i) Diarylbutadiene und<br>(j) aminosubstituierten Hydroxybenzophenone als textilaffine UV-Absorber. |

Weiterhin betrifft die vorliegende Erfindung ein Verfahren zum Schutz der menschlichen Haut vor schädigender UV-Strahlung und ein Verfahren zum Schutz von gefärbtem textilem Material vor Verblassung der Farbe, weiterhin ein Verfahren zur Erhöhung des UV-Schutzfaktors UPF von textilem Material, weiterhin eine Textilwaschmittel- sowie eine Textilwäschevor- und Textilwäschenachbehandlungs-Formulierung, welche diese faseraffine UV-Absorber-Mischung enthalten und weiterhin textiles Material, welches diese faseraffine UV-Absorber-Mischung enthält, eine Mischung aus

| | | |
|---|---|---|
| (A) | 10 bis 90 Gew.-% | mindestens eines $C_6$- bis $C_{18}$-Alkylesters oder $C_5$- bis $C_8$-Cycloalkylesters der 2-Cyano-,3-diphenylacrylsäure und |
| (B) | 90 bis 10 Gew.-% | mindestens einer weiteren Verbindung, welche mindestens ein UV-Absorptionsmaximum im Bereich von 280 bis 450 nm aufweist und ausgewählt ist aus der Gruppe der<br>(a) Phenylbenzotriazole,<br>(i) Diarylbutadiene und<br>(j) aminosubstituierten Hydroxybenzophenone, sowie die Verwendung von UV-Absorbern aus der Gruppe der Diarylbutadiene und der aminosubstituierten Hydroxybenzophenone als textilfaseraffine UV-Absorber in auf textiles Material aufgebrachter Form zum Schutz der menschlichen Haut vor schädigender UV-Strahlung und/oder zum Schutz von gefärbtem textilem Material vor Verblassung der Farbe. |

Die schädigenden Auswirkungen der UV-Strahlung des Sonnenlichtes auf die menschliche Haut beschränken sich nicht nur auf eine vorzeitige Hautalterung und die Bildung von Hauterythemen (Hautrötungen, Sonnenbrand). Wird die Haut zu lange und zu intensiv der UV-Strahlung ausgesetzt, so erhöht sich zusätzlich die Gefahr, an Hautkrebs zu erkranken. Vornehmlich verantwortlich für die Hautrötung und das erhöhte Hautkrebsrisiko ist der UV-B-Anteil der UV-Strahlung, d.h. der Bereich von ca. 280 bis ca. 315 nm. Das Maximum der Erythemen-Wirkung liegt bei 308 nm.

Textilien absorbieren UV-Strahlung und schützen somit als physikalische Barriere die Haut vor den schädigenden Einflüssen des Sonnenlichtes ("textiler Hautschutz"). Allerdings ist die hautschützende Wirkung der Textilien von vielen Faktoren wie Faserart, Gewebekonstruktion, Stoffgewicht, Farbe, Feuchtigkeitsgehalt oder Art der Ausrüstung bzw. Veredlung abhängig. So bietet gerade Sommerkleidung in Form von leichten und hellen Baumwolltextilien nur einen schwachen und damit unzulänglichen Schutz vor UV-Strahlung.

Eine zu hohe Dosis an UV-Strahlung kann nicht nur Hautschäden verursachen, sie ist auch maßgeblich verantwortlich für die durch Sonnenlicht induzierte Verblaßung von farbigen Textilien. Deshalb besteht ein hohes Interesse, sowohl farbige Textilien als auch die menschliche Haut vor den schädigenden Einwirkungen der UV-Strahlung zu schützen.

Bislang werden hauptsächlich übliche optische Aufheller zur Veredlung und zum Schutz der Textilien selbst und auch zum textilen Hautschutz eingesetzt, insbesondere solche auf Stilben- und Triazin-Basis wie beispielsweise in der EP-A 682 145, GB-A 2 313 375 oder der EP-A 728 749 beschrieben. Die Mittel sind aber in ihrer Wirkung noch verbesserungsbedürftig und weisen eine Reihe von Nachteilen auf. Wesentliche Nachteile sind ihre schlechte Formulierbarkeit und ihre mangelnde Löslichkeit im jeweiligen Anwendungsmedium.

Aus der WO 97/44422 ist bekannt, daß Sonnenschutzmittel insbesondere aus der Gruppe der Phenylbenzotriazole, Dibenzoylmethane, p-Aminobenzoesäureester, Zimtsäureester, Salicylsäureester, stickstofffreien 2-Hydroxybenzophenone, Phenylbenzimidazole und 2-Cyano-3,3-diphenylacrylsäureester sowie Mischungen hieraus - allerdings ohne Hinweise auf die jeweiligen Mischungsverhältnisse - gefärbtes textiles Material vor dem Verblassen der Farbe schützen. Als Beispiele für 2-Cyano-3,3-diphenylacrylsäureester werden der 2-Ethyl- und der 2-Ethylhexylester genannt. Experimentelle Beispiele werden mit 2-Ethylhexyl-4-methoxycinnamat (Parsol® MCX) und 2-(2'-Hydroxy-3'. 5'-di-tert.-amylphenyl)-2H-benzotriazol (Tinuvin® 328) beschrieben.

In der WO 96/03486 werden im wesentlichen die gleichen Sonnenschutzmittel, wie sie in der WO 97/44422 genannt werden, gleichermaßen als Mittel zum Schutz von gefärbtem textilem Material vor dem Verblassen der Farbe beschrieben. Diese Mittel sind aber in ihrer Wirkung ebenfalls noch verbesserungsbedürftig und weisen eine Reihe von Nachteilen auf.

Aufgabe der vorliegenden Erfindung war es, in ihrer Wirkung verbesserte faseraffine UV-Absorber-Systeme bereitzustellen, welche die Nachteile des Standes der Technik nicht mehr aufweisen.

Demgemäß wurde die eingangs definierte Mischung aus den Verbindungen (A) und (B) gefunden.

Der $C_6$- bis $C_{18}$-Alkoholrest in den Estern (A) kann linear oder ein- oder mehrfach verzweigt sein, er kann natürlichen oder synthetischen Ursprungs ein. Derartige Alkohole können beispielsweise Fettalkohole, Oxo-Alkohole, Ziegler-Alkohole oder Guerbet-Alkohole sein. Typische Beispiele für derartige Verbindungen (A) sind der n-Hexyl-, n-Heptyl-, n-Octyl, 2-Ethylhexyl-, n-Nonyl-, iso-Nonyl-, n-Decyl, n-Dodecyl-, n-Tridecyl-, iso-Tridecyl-, n-Tetradecyl-, n-Pentadecyl-, n-Hexadecyl-, n-Heptadecyl-, n-Octadecyl oder Eicosylester. Es können auch ungesättigte Alkoholreste wie der Oleyl-, Linolyl- oder der Linolenyl-Rest auftreten. Es können auch Mischungen derartiger Ester vorliegen.

Als $C_5$- bis $C_8$-Cycloalkanolreste in den Estern (A) kommen beispielsweise Cycolpentyl-, 2- oder 3-Methylcyclopentyl-, Cylcohexyl-, 2-, 3- oder 4-Methylcyclohexyl-, Dimethylcyclohexyl-, Cycloheptyl- oder Cyclooctyl-Reste in Betracht.

Bevorzugt werden lineare oder ein- oder mehrfach verzweigte $C_8$-bis $C_{13}$-Alkylesters der 2-Cyano-3,3-diphenylacrylsäure. Der entsprechende 2-Ethylhexylester ist als Uvinul® N-539 T der BASF Aktiengesellschaft im Handel.

Von großer Bedeutung ist das Mischungsverhältnis der Verbindungen (A) mit den Verbindungen (B). Jede der beiden Komponenten muß zu einem Anteil von mindestens 10 Gew.-% in der erfindungsgemäßen Mischung vorliegen. Bevorzugt wird eine Mischung aus 20 bis 80 Gew.-% (A) und 80 bis 20 Gew.-% (B), insbesondere aus 30 bis 70 Gew.-% (A) und 70 bis 30 Gew.-% (B), vor allem aus 40 bis 60 Gew.-% (A) und 60 bis 40 Gew.-% (B).

Als Verbindungen (B) können meist handelsübliche Sonnenschutzmittel verwendet werden, welche mindestens ein UV-Absorptionsmaximum im Bereich von 280 bis 450 nm aufweisen und aus den in Anspruch 1 bzw. Anspruch 14 genannten Gruppen ausgewählt sind.

In einer bevorzugten Ausführungsform enthält die erfindungsgemäße Mischung solche Verbindungen (B), die mindestens ein UV-Absorptionsmaximum im Bereich von 330 bis 380 nm aufweisen.

In einer weiteren bevorzugten Ausführungsform enthält die erfindungsgemäße Mischung solche Verbindungen (B), die mindestens ein UV-Absorptionsmaximum im Bereich von 280 bis 315 nm (UV-B-Bereich) mit einem $E^1_1$-Wert von mindestens 200, insbesondere von mindestens 250, und mindestens ein UV-Absorptionsmaximum im Bereich von 315 bis 400 nm (UV-A-Bereich) mit einem $E^1_1$-Wert von mindestens 200, insbesondere von mindestens 250, aufweisen.

Sonnenschutzmittel wie die Verbindungen (B) weisen im UV-Absorptionsspektrum eine oder mehrere Banden auf. Unter UV-Absorptionsmaxima sind hier die zu den entsprechenden lokalen oder absoluten Maxima im UV-Spektrum der jeweiligen Verbindung gehörigen Banden, gemessen in üblichen organischen Lösungsmitteln wie Dichlormethan oder Methanol bei Raumtemperatur, zu verstehen.

Der $E^1_1$-Wert bezeichnet die Extinktion der Verbindung (B), welche in Lösung bei einer Konzentration von 1 Gew.-% und einer Schichtdicke von 1 cm gemessen wird. Als Lösungsmittel wird hierbei üblicherweise Dichlormethan eingesetzt, die Verwendung anderer für derartige UV-Messungen üblicher Lösungsmittel erbringt keine grundsätzlich verschiedenen Werte.

In einer weiteren bevorzugten Ausführungsform enthält die erfindungsgemäße Mischung solche Verbindungen (A) und solche Verbindungen (B), die im Temperaturbereich von 10 bis 35°C ohne Zusatz von Lösungs- oder Verdünnungsmitteln eine gemeinsame homogene flüssige Phase ausbilden. Diese gemeinsame homogene flüssige Phase kann meist als Lösung der einen Komponente in der anderen aufgefaßt werden. Der Vorteil solcher homogenen Mischungen ist insbesondere deren einfache Herstellbarkeit und deren bessere Applizierbarkeit.

In einer weiteren bevorzugten Ausführungsform enthält die erfindungsgemäße Mischung solche Verbindungen (B), die einen n-Octanol/Wasser-Verteilungskoeffizienten log P von mindestens 1,9, insbesondere von mindestens 2,5, vor allem von mindestens 3,3 aufweisen. Log P kann experimentell bestimmt oder berechnet werden. Beide Verfahren werden in Chemical Reviews Volume 71, No. 5, Seiten 52-5-616 (1971) beschrieben.

Die Verbindungen (B) werden ausgewählt aus der Gruppe der

(a) Phenylbenzotriazole,

(b) Dibenzoylmethane,

(i) Diarylbutadiene und

(j) aminosubstituierten Hydroxybenzophenone.

Typische UV-absorbierende Phenylbenzotriazole (a) sind:

2,2'-Hydroxy-5-methylphenyl-benzotriazol,
2,2'-Hydroxy-5-tert.-octylphenyl-benzotriazol,
2-Hydroxy-3-sec.-butyl-5-tert.-butyl-benzotriazol (Tinuvin® 350),
2-Hydroxy-3-dodecyl-5-methylphenyl-benzotriazol (Tinuvin® 571),
2-(2H-Benzotriazol-2-yl)-4-methylphenol (Tinuvin® P),
2-(2H-Benzotriazol-2-yl)-4-n-octylphenyl (Tinuvin® 329),
2-(2H-Benzotriazol-2-yl)-4,6-di(2'-phenylisopropyl)-phenol (Tinuvin® 234),
2-(2H-Benzotriazol-2-yl)-4,6-di(tert.-butyl)-phenol (Tinuvin® 320) ,
2-(6-Chlor-2H-benzotriazol-2-yl)-4-methyl-6-tert.-butylphenol (Tinuvin® 326),
2-(6-Chlor-2H-benzotriazol-2-yl)-2,6-di-tert.-butylphenol (Tinuvin® 327),
2-(2'-Hydroxy-3',5'-di-tert.-amylphenyl)-2H-benzotriazol (Tinuvin® 328),
Mischung aus β-[3-(2H-Benzotriazol-2-yl)-5-tert.-butyl-4-hydroxyphenyl]-propionsäure-polyoxyethylenglykolester und Polyoxyethylenglykol-bis-β-[3-(2H-Benzotriazol-2-yl)-5-tert.-butyl-4-hydroxyphenyl]-propionat mit einem durchschnittlichen Molekulargewicht >600 (Tinuvin® 1130),
Cocoyl-2-[2'-Hydroxy-3'-(cocoyl-dimethyl-butanoat)-5'-methylphenyl]-benzotriazol,
Cocoyl-3-[3'-(2H-benzotriazol-2'-yl)-5-tert.-butyl-4'-hydroxyphenyl]-propionat,
2,2'-Methylen-bis-[6-(2H-benzotriazol-2-yl)-4-(1,1,3,3-tetramethylbutyl)]-phenol (Tinuvin® 360),
2-(2H-1,2,3-Benzotriazol-2-yl)-4-methyl-6-(2-methyl-3-{1,3,3,3-tetramethyl-1-[(trimethylsilyl)oxy]disiloxanyl}propyl)-phenol.

Typische UV-absorbierende Dibenzoylmethane (b) sind:

3-(4-Isopropyl-phenyl)-3-phenyl-propan-1,3-dion (Eusolex® 8020),
1-(4-tert.-Butylphenyl)-3-(4-methoxyphenyl)-propan-1,3-dion (Uvinul® BMBM),
1,3-Bis-(4-methoxyphenyl)-propan-1,3-dion.

Typische UV-absorbierende Diarylbutadiene (i) sind insbesondere 4,4-Diarylbutadiene der Formel I, wie sie in der DE-A 198 28 463 für kosmetische und pharmazeutische Zubereitungen beschrieben sind:

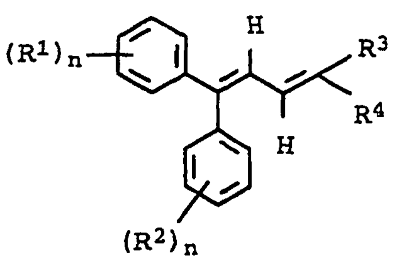

I

in der das Diensystem in der Z,Z; Z,E; E,Z oder E,E Konfiguration oder einer Mischung davon vorliegt und in der die Variablen unabhängig voneinander folgende Bedeutung haben:

$R^1$ und $R^2$ Wasserstoff, $C_1$-$C_{20}$-Alkyl, $C_2$-$C_{10}$-Alkenyl, $C_3$-$C_{10}$-Cycloalkyl, $C_3$-$C_{10}$-Cycloalkenyl, $C_1$-$C_{12}$-Alkoxy, $C_1$-$C_{20}$-Alkoxycarbonyl, $C_1$-$C_{12}$-Alkylamino, $C_1$-$C_{12}$-Dialkylamino, Aryl, Heteroaryl, gegebenenfalls substituiert, wasserlöslich machende Substituenten, ausgewählt aus der Gruppe bestehend aus Carboxylat-, Sulfonat- oder Ammoniumresten;

$R^3$ Wasserstoff, $COOR^5$, $COR^5$, $CONR^5R^6$, CN;

$R^4$ $COOR^6$, $COR^6$, $CONR^5R^6$;

$R^5$ Wasserstoff, $[X]_o$-$R^7$, $C_1$-$C_6$-Alkylen-$SO_3Y$, $C_1$-$C_6$-Alkylen-$PO_3Y$, $C_1$-$C_6$-Alkylen-$N(R^8)_3^+$ $A^-$;

$R^6$ $[X]_o$-$R^7$, $C_1$-$C_6$-Alkylen-$SO_3Y$, $C_1$-$C_6$-Alkylen-$PO_3Y$, $C_1$-$C_6$-Alkylen-$N(R^8)_3^+$ A-;

X -$CH_2$-$CH_2$-Z-, -$CH_2$-$CH_2$-$CH_2$-Z-, -$CH(CH_3)$-$CH_2$-Z-, -$CH_2$-$CH_2$-$CH_2$-$CH_2$-Z-, -$CH_2$-$CH(CH_2$-$CH_3)$-Z-;

A Cl, Br, J, $SO_4R^9$;

Y Wasserstoff, $Na^+$, $K^+$, $Mg^{2+}$, Ca2+, $Li^+$, $Al^{3+}$, $N(R^8)_4^+$;

Z O, NH;

$R^7$ und $R^8$ Wasserstoff, $C_1$-$C_6$-Alkyl, $C_2$-$C_6$-Alkenyl, $C_1$-$C_6$-Acyl;

$R^9$ Wasserstoff, $C_1$-$C_6$-Alkyl, $C_2$-$C_6$-Alkenyl;

n 1 bis 3;

o 1 bis 150

Bezüglich der näheren Spezifizierung der Strukturen der 4,4-Diarylbutadiene der Formel I und Beispiele für solche Verbindungen I wird in diesem Zusammenhang ausdrücklich auf die Offenbarung der DE-A 198 28 463 verwiesen. Als ein typisches Beispiel für einen Vertreter einer solchen Verbindung (i) soll 1,1-Bis-(neonentyloxycarbonyl)-4,4-diphenyl-1,3-butadien genannt werden.

Typische UV-absorbierende aminosubstituierte Hydroxybenzophenone (j) sind insbesondere solche der Formel II, wie sie in der deutschen Patentanmeldung Az. 199 17 906.9 für kosmetische und pharmazeutische Zubereitungen beschrieben sind:

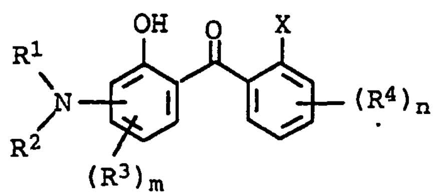

II

in der die Variablen unabhängig voneinander folgende Bedeutung haben:

$R^1$ und $R^2$ Wasserstoff, $C_1$-$C_{20}$-Alkyl, $C_2$-$C_{10}$-Alkenyl, $C_3$-$C_{10}$-Cycloalkyl, $C_3$-$C_{10}$-Cycloalkenyl, wobei die Substituenten $R^1$ und $R^2$ gemeinsam mit dem Stickstoffatom, an dem sie gebunden sind, einen 5- oder 6-Ring bilden können;

$R^3$ und R4 $C_1$-$C_{20}$-Alkyl, $C_2$-$C_{10}$-Alkenyl, $C_3$-$C_{10}$-Cycloalkyl, $C_3$-$C_{10}$-Cycloalkenyl, $C_1$-$C_{12}$-Alkoxy, $C_1$-$C_{20}$-Alkoxycarbonyl, $C_1$-$C_{12}$-Alkylamino, $C_1$-$C_{12}$-Dialkylamino, Aryl, Heteroaryl, gegebenenfalls substituiert, wasserlöslich machende Substituenten, ausgewählt aus der Gruppe bestehend aus einer Nitrilgruppe, Carboxylat-, Sulfonat- oder Ammoniumresten;

X Wasserstoff, $COOR^5$, $CONR^6R^7$;

$R^5$ bis $R^7$ Wasserstoff, $C_1$-$C_{20}$-Alkyl, $C_2$-$C_{10}$-Alkenyl, $C_3$-$C_{10}$-Cycloalkyl, $C_3$-$C_{10}$-Cycloalkenyl, -$(Y$-$O)_o$-Z, Aryl;

Y -$(CH_2)_2$-, -$(CH_2)_3$-, -$(CH_2)_4$-, -$CH(CH_3)$-$CH_2$-;

Z -$CH_2$-$CH_3$, -$CH_2$-$CH_2$-$CH_3$, -$CH_2$-$CH_2$-$CH_2$-$CH_3$, -$CH(CH_3)$-$CH_3$;

m 0 bis 3;

n 0 bis 4;

o 1 bis 20

Bezüglich der näheren Spezifizierung der Strukturen der aminosubstituierten Hydroxybenzophenone der Formel II und Beispiele für solche Verbindungen II wird in diesem Zusammenhang ausdrücklich auf die Offenbarung der deutschen Patentanmeldung Az. 199 17 906.9 verwiesen. Als ein typisches Beispiel für einen Vertreter einer solchen Verbindung (j) soll 2-(4'-Diethylamino-2'-hydroxybenzoyl)-benzoesäure-n-hexylester genannt werden.

Die UV-Absorber aus der Gruppe der Diarylbutadiene (i) und der aminosubstituierten Hydroxybenzophenone (j) eignen sich jeweils auch alleine als textilfaseraffine UV-Absorber in auf textiles Material aufgebrachter Form zum Schutz der menschlichen Haut vor schädigender UV-Strahlung und/oder zum Schutz von gefärbtem textilem Material vor Verblassung der Farbe. Die nachfolgenden Aussagen in Hinblick auf die Anwendungen der erfindungsgemäßen Mischung gelten gleichermaßen auch für die Diarylbutadiene (i) und die aminosubstituierten Hydroxybenzophenone (j) allein.

Vertreter der Verbindungen (B) des Typs (a) und (b) sind im übrigen in der WO 97/44422 und der WO 96/03486 beschrieben.

Neben den Verbindungen des Typs (a) (b), (i) oder (j) können als Verbindungen (B) weiterhin beispielsweise auch noch die folgenden verwendet werden:

3-(4-Methylbenzyliden)-bornan-2-on (Eusolex® 6300),
5-(3,3-Dimethyl-2-norbornyliden)-3-penten-2-on,
3-Benzyliden-bornan-2-on,
Digalloyl-trioleat,
2-Hydroxy-1,4-naphthalendion,
5-Methyl-2-phenylbenzoxazol,
Dibenzaldehydamin,
Dianisoylmethan,
Methyleugenol,
2-Amino-6-hydroxypurin,
N-(4-Ethoxycarbonylphenyl)-N'-methyl-N'-phenylformamidin (Givosorb® UV1),
N-(4-Ethoxycarbonylphenyl)-N'-ethyl-N'-phenylformamidin (Givosorb® UV2),
3-(4'-Methylbenzyliden)-campher (Uvinul® MBC95),
$N^1$-(2-Ethoxyphenyl)-$N^2$-(2-ethylphenyl)-ethandiamid,
$N^1$-(2-Ethoxyphenyl)-$N^2$-(4-dodecaphenyl)-ethandiamid,
2-Ethylhexyl-2-cyano-(3-oxo-2,3-dihydro-1H-isoindol-1-yliden)-ethanoat,
1,1-Dicyano-2-(4-butyloxy)phenyl-2-phenyl-ethen.

Bisweilen kann es von Vorteil sein, wenn die erfindungsgemäße Mischung aus den Komponenten (A) und (B) Antioxidantien und/oder Radikalfänger enthält oder zusammen mit solchen in ihrer Verwendung als textilfaseraffine UV-Absorber eingesetzt wird.

Zu den Antioxidantien und Radikalfängern, die hier zur Anwendung gelangen können, zählen alle üblichen derartigen Verbindungen, insbesondere jedoch:

- substituierte Phenole, Hydrochinone und Brenzcatechine wie 2,6-Di-tert.-butylphenol, 2,4,6-Tri-t-butylphenol, 2,4-Dimethyl-6-tert.-butylphenol, 2,6-Di-tert.-butyl-4-methylphenol, 2,6-Di-tert.-butyl-4-methoxyphenol, 3-tert.-Butyl-4-methoxyphenol, 2,2'-Methylenbis(4-methyl-6-tert.-butylphenol), Propyl-,Octyl- und Dodecylgallat oder tert. -Butylhydrochinon
- Ascorbin-, Milch-, Citronen- und Weinsäure und deren Salze
- Tocopherol (Acetyl-$\alpha$-tocopherol)
- Diazobicyclo[2,2,2]octan
- übliche aromatische Amine
- übliche organische Sulfide
- übliche sterisch gehinderte Amine wie Uvinul® 4049H, Uvinul 4050H und Uvinul 5050H (vertrieben von BASF Aktiengesellschaft)

Die erfindungsgemäße Mischung aus den Komponenten (A) und (B) eignet sich in hervorragender Weise als textilfaseraffine UV-Absorber-Mischung. Zum einen schützt die erfindungsgemäße Mischung in auf textiles Material aufgebrachter Form die menschliche Haut vor schädigender UV-Strahlung. Zum anderen schützt die erfindungsgemäße Mischung gefärbtes textiles Material vor Verblassung der Farbe. Vorzugsweise treten beide Schutzeffekte gleichzeitig auf.

Textiles Material, worauf die erfindungsgemäße Mischung der vorliegenden Erfindung aufziehen und dort ihre Schutzwirkung entfalten kann, umfaßt insbesondere Bekleidungsartikel, d.h. Textilien, die auf der menschlichen Haut getragen werden, aber auch Haus-und Gartenartikel aus oder mit gefärbten Textilien wie Markisen und Sonnenschirme, die intensiver Sonnenbestrahlung ausgesetzt sind. Vorzugsweise besteht dieses zu schützende textile Material aus Cellulose (Baumwolle) oder enthält Cellulose, beispielsweise sind hier Bekleidungstextilien aus Baumwolle oder Baumwoll-Polyester-Mischungen von Interesse.

Gegenstand der vorliegenden Erfindungen ist auch ein Verfahren zum Schutz der menschlichen Haut vor schädigender UV-Strahlung, welches dadurch gekennzeichnet ist, daß man die erfindungsgemäße Mischung auf textiles Material bei der Textilausrüstung, also bei der Herstellung der Textilien, aufbringt.

Gegenstand der vorliegenden Erfindung ist weiterhin auch ein Verfahren zum Schutz der menschlichen Haut vor schädigender UV-Strahlung, welches dadurch gekennzeichnet ist, daß man die erfindungsgemäße Mischung auf textiles Material bei der Textilwäsche und/oder der Textilwäschevor- oder Textilwäschenachbehandlung aufbringt.

Gegenstand der vorliegenden Erfindung ist weiterhin auch ein Verfahren zum Schutz von gefärbtem textilem Material vor Verblassung der Farbe, welches dadurch gekennzeichnet ist, daß man die erfindungsgemäße Mischung auf textiles Material bei der Textilausrüstung, also bei der Herstellung der Textilien, aufbringt.

Gegenstand der vorliegenden Erfindung ist weiterhin auch ein Verfahren zum Schutz von gefärbtem textilem Material vor Verblassung der Farbe, welches dadurch gekennzeichnet ist, daß man die erfindungsgemäße Mischung auf textiles Material bei der Textilwäsche und/oder der Textilwäschevor- oder Textilwäschenachbehandlung aufbringt.

Gegenstand der vorliegenden Erfindung ist weiterhin auch ein Verfahren zur Erhöhung des UV-Schutzfaktors UPF von textilem Material, welches dadurch gekennzeichnet ist, daß man die erfindungsgemäße Mischung auf textiles Material bei der Textilausrüstung, also bei der Herstellung der Textilien, aufbringt.

Gegenstand der vorliegenden Erfindung ist weiterhin auch ein Verfahren zur Erhöhung des UV-Schutzfaktors UPF von textilem Material, welches dadurch gekennzeichnet ist, daß man die erfindungsgemäße Mischung auf textiles Material bei der Textilwäsche und/oder der Textilwäschevor- oder Textilwäschenachbehandlung aufbringt.

Der UV-Schutzfaktor UPF ("Ultraviolet Radiation Protection Factor") von Textilien wird gemäß der australischen/neuseeländisch, Norm AS/NZS 4399:1996 mittels einer in vitro-Methode gestimmt. Gemessen wird die UV-Durchlässigkeit des textilen Objektes. Aus der spektralen Transmission läßt sich anhand der nachfolgenden Gleichung der Schutzfaktor direkt ermitteln:

$$UPF = \frac{\int_{\lambda = 280\,nm}^{400\,nm} S_\lambda \times E_\lambda \times d\lambda}{\int_{\lambda = 280\,nm}^{400\,nm} S_\lambda \times E_\lambda \times T_\lambda \times d\lambda}$$

wobei:

$S_\lambda$= Spektraleinstrahlung der Sonne im UV-Bereich bei der Wellenlänge $\lambda$
$E_\lambda$ = spektrale Erythemwirksamkeit der UV-Strahlung bei de Wellenlänge $\lambda$
$T_\lambda$ = spektrale Durchlässigkeit des Textilen Objektes bei Wellenlänge $\lambda$

Die erfindungsgemäße Mischung kann bei der Ausrüstung des texti len Materials, also bei der Herstellung der Textilien, oder bei der Pflege des fertigen textilen Gegenstandes, also bei der Tex tilwäsche und/oder der Textilwäschevor- oder Textilwäschenachbe handlung, aufgebracht werden.

Unter Textilausrüstung ("Textilveredlung") versteht man die Arbeitsprozesse bei der Herstellung von Textilien, bei denen der Gebrauchswert der Textilien gesteigert wird und durch eine vorteilhafte Gestaltung ihrer äußeren Eigenschaften die Textilien verschönert werden. Typische Prozesse zur Gebrauchswertsteigeru: sind die Pflegeleichtausrüstung, das Knitterfestmachen und die Krumpfechtausrüstung. Typische Prozesse zur Gestaltung der äuße ren Eigenschaften sind das Färben, das Bleichen, das Bedrucken und das Mercerisieren. Bei der Ausrüstung insbesondere von Stüc ware werden in der Regel Appreturen eingesetzt, welche die veredelnden Mittel enthalten und denen vorzugsweise auch die erfin dungsgemäße Mischung im Sinne der vorliegenden Erfindung zugesetzt wird.

Gegenstand der vorliegenden Erfindung ist auch eine Textilwaschmittel-Formulierung, welche 0,01 bis 20 Gew.-%, insbesondere 0,1 bis 10 Gew.-%, vor allem 0,1 bis 5 Gew.-%, einer erfindungsgemäßen Mischung aus den Komponenten (A) und (B) neben den sonstigen üblichen Bestandteilen enthält. Dabei kann die Zugabe der Komponenten (A) und (B) im Prinzip separat in die Formulierung erfolgen oder es kann die vorgefertigte Mischung aus (A) und (B) in die Formulierung eingearbeitet werden; letztere Methode ist in vielen Fälle die günstigere, insbesondere wenn (A) und (B) im Temperaturbereich von 10 bis 35°C ohne Zusatz von Lösungs- oder Verdünnungsmitteln eine gemeinsame homogene flüssige Phase ausbilden.

Die erfindungsgemäße Textilwaschmittel-Formulierung enthält in der Regel als sonstige übliche Bestandteile

(B) 1 bis 60 Gew.-% anorganische Builder auf Basis von kristallinen oder amorphen Alumosilicaten, kristallinen oder amorphen Silicaten, Carbonaten oder Phosphaten,

(C) 0,5 bis 40 Gew.-% anionische Tenside und

(D) 0,5 bis 40 Gew.-% nichtionische Tenside.

Geeignete anorganische Builder (B) sind vor allem kristalline oder amorphe Alumosilicate mit ionenaustauschenden Eigenschaften wie insbesondere Zeolithe. Verschiedene Typen von Zeolithen sind geeignet, insbesondere Zeolithe A, X, B, P, MAP und HS in ihrer Na-Form oder in Formen, in denen Na teilweise gegen andere Kationen wie Li, K, Ca, Mg oder Ammonium ausgetauscht ist. Geeignete Zeolithe sind beispielsweise beschrieben in EP-A 038591, EP-A 021491, EP-A 087035, US-A 4604224, GB-A 2013259, EP-A 522726, EP-A 384070 und WO-A 94/24251.

Geeignete kristalline Silicate (B) sind beispielsweise Disilicate oder Schichtsilicate, z.B. $\delta$-$Na_2Si_2O_5$ oder $\beta$-$Na_2Si_2O_5$ (SKS 6 bzw. SKS 7, Hersteller: Hoechst). Die Silicate können in Form ihrer Alkalimetall-, Erdalkalimetall- oder Ammoniumsalze eingesetzt werden, vorzugsweise als Na-, Li- und Mg-Silicate.

Amorphe Silicate wie beispielsweise Natriummetasilicat, welches eine polymere Struktur aufweist, oder amorphes Disilicat (Britesil® H 20 Hersteller: Akzo) sind ebenfalls verwendbar.

Geeignete anorganische Buildersubstanzen (B) auf Carbonat-Basis sind Carbonate und Hydrogencarbonate. Diese können in Form ihrer Alkalimetall-, Erdalkalimetall- oder Ammoniumsalze eingesetzt werden. Vorzugsweise werden Na-, Li- und Mg-Carbonate bzw. -Hydrogencarbonate, insbesondere Natriumcarbonat und/oder Natriumhydrogencarbonat, eingesetzt.

Übliche Phosphate als anorganische Builder (B) sind Polyphosphate wie z.B. Pentanatriumtriphosphat.

Die genannten Komponenten (B) können einzeln oder in Mischungen untereinander eingesetzt werden.

Die Komponente (B) liegt in der erfindungsgemäßen Textilwaschmittel-Formulierung vorzugsweise in einer Menge von 5 bis 50 Gew.-%, insbesondere 10 bis 45 Gew.-%, vor.

In einer bevorzugten Ausführungsform enthält die erfindungsgemäße Textilwaschmittel-Formulierung keine Builder auf Phosphat-Basis oder solche nur bis maximal 5 Gew.-%, insbesondere nur bis maximal 2 Gew.-%.

Geeignete anionische Tenside (C) sind beispielsweise Fettalkoholsulfate von Fettalkoholen mit 8 bis 22, vorzugsweise 10 bis 18 Kohlenstoffatomen, z.B. $C_9$- bis $C_{11}$-Alkoholsulfate, $C_{12}$- bis $C_{14}$-Alkoholsulfate, $C_{12}$-$C_{18}$-Alkoholsulfate, Laurylsulfat, Cetylsulfat, Myristylsulfat, Palmitylsulfat, Stearylsulfat und Talgfettalkoholsulfat.

Weitere geeignete anionische Tenside sind sulfatierte ethoxylierte $C_8$- bis $C_{22}$-Alkohole (Alkylethersulfate) bzw. deren lösliche Salze. Verbindungen dieser Art werden beispielsweise dadurch hergestellt, daß man zunächst einen $C_8$- bis $C_{22}$-, vorzugsweise einen $C_{10}$- bis $C_{18}$-Alkohol z.B. einen Fettalkohol, alkoxyliert und das Alkoxylierungsprodukt anschließend sulfatiert. Für die Alkoxylierung verwendet man vorzugsweise Ethylenoxid, wobei man pro Mol Alkohol 1 bis 50, vorzugsweise 1 bis 20 Mol Ethylenoxid einsetzt. Die Alkoxylierung der Alkohole kann jedoch auch mit Propylenoxid allein und gegebenenfalls Butylenoxid durchgeführt werden. Geeignet sind außerdem solche alkoxylierte $C_8$- bis $C_{22}$-Alkohole, die Ethylenoxid und Propylenoxid oder Ethylenoxid und Butylenoxid oder Ethylenoxid und Propylenoxid und Butylenoxid enthalten. Die alkoxylierten $C_8$- bis $C_{22}$-Alkohole können die Ethylenoxid-, Propylenoxid- und Butylenoxideinheiten in Form von Blöcken oder in statistischer Verteilung enthalten. Je nach Art des Alkoxylierungskatalysators kann man Alkylethersulfate mit breiter oder enger Alkylenoxid-Homologen-Verteilung erhalten.

Weitere geeignete anionische Tenside sind Alkansulfonate wie $C_8$-bis $C_{24}$-, vorzugsweise $C_{10}$- bis $C_{18}$-Alkansulfonate sowie Seifen wie beispielsweise die Na- und K-Salze von $C_8$- bis $C_{24}$-Carbonsäuren.

Weitere geeignete anionische Tenside sind lineare $C_8$- bis $C_{20}$-Alkylbenzolsulfonate ("LAS"), vorzugsweise lineare $C_9$- bis $C_{13}$-Alkylbenzolsulfonate und -Alkyltoluolsulfonate.

Weiterhin eignen sich als anionische Tenside (C) noch $C_8$- bis $C_{24}$-Olefinsulfonate und -disulfonate, welche auch Gemische aus Alken- und Hydroxyalkansulfonaten bzw. -disulfonate darstellen können, Alkylestersulfonate, sulfonierte Polycarbonsäuren, Alkylglycerinsulfonate, Fettsäureglycerinestersulfonate, Alkylphenolpolyglykolethersulfate, Paraffinsulfonate mit ca. 20 bis ca. 50 C-Atomen (basierend auf aus natürlichen Quellen gewonnenem Paraffin oder Paraffingemischen), Alkylphosphate, Acylisethionate, Acyltaurate, Acylmethyltaurate, Alkylbernsteinsäuren, Alkenylbernsteinsäuren oder deren Halbester oder Halbamide, Alkylsulfobernsteinsäuren oder deren Amide, Mono- und Diester von Sulfobernsteinsäuren, Acylsarkosinate, sulfatierte Alkylpolyglucoside, Alkylpolyglykolcarboxylate sowie Hydroxyalkylsarkosinate.

Die anionischen Tenside werden dem Waschmittel vorzugsweise in Form von Salzen zugegeben. Geeignete Kationen in diesen Salzen sind Alkalimetallionen wie Natrium, Kalium und Lithium und Ammoniumsalze wie z.B. Hydroxyethylammonium-, Di(hydroxyethyl)ammonium- und Tri(hydroxyethyl)ammoniumsalze.

Die Komponente (C) liegt in der erfindungsgemäßen Textilwaschmittel-Formulierung vorzugsweise in einer Menge von 1 bis 30 Gew.-%, vor allem 3 bis 25 Gew.%, insbesondere 5 bis 15 Gew.-% vor. Werden $C_9$- bis $C_{20}$-linear-Alkyl-benzolsulfonate (LAS) mitverwendet, kommen diese üblicherweise in einer Menge bis zu 10 Gew.-%, insbesondere bis zu 8 Gew.-%, zum Einsatz.

Man kann einzelne anionische Tenside oder eine Kombination unterschiedlicher Aniontenside einsetzen. Es können anionische Tenside aus nur einer Klasse zum Einsatz gelangen, beispielsweise nur Fettalkoholsulfate oder nur Alkylbenzolsulfonate, man kann aber auch Tensidmischungen aus verschiedenen Klassen verwenden, z.B. eine Mischung aus Fettalkoholsulfaten und Alkylbenzolsulfonaten.

Als nichtionische Tenside (D) eignen sich beispielsweise alkoxylierte $C_8$- bis $C_{22}$-Alkohole wie Fettalkoholalkoxylate oder Oxoalkoholalkoxylate. Die Alkoxylierung kann mit Ethylenoxid, Propylenoxid und/oder Butylenoxid durchgeführt werden. Als Tenside einsetzbar sind hierbei sämtliche alkoxylierten Alkohole, die mindestens zwei Moleküle eines vorstehend genannten Alkylenoxids addiert enthalten. Auch hierbei kommen Blockpolymerisate von Ethylenoxid, Propylenoxid und/oder Butylenoxid in Betracht oder Anlagerungsprodukte, die die genannten Alkylenoxide in statistischer Verteilung enthalten. Pro Mol Alkohol verwendet man 2 bis 50, vorzugsweise 3 bis 20 Mol mindestens eines Alkylenoxids. Vorzugsweise setzt man als Alkylenoxid Ethylenoxid ein. Die Alkohole haben vorzugsweise 10 bis 18 Kohlenstoffatome. Je nach Art des Alkoxylierungskatalysators kann man Alkoxylate mit breiter oder enger Alkylenoxid-Homologen-Verteilung erhalten.

Eine weitere Klasse geeigneter nichtionischer Tenside sind Alkylphenolalkoxylate wie Alkylphenolethoxylate mit $C_6$ bis $C_{14}$-Alkylketten und 5 bis 30 Mol Alkylenoxideinheiten.

Eine andere Klasse nichtionischer Tenside sind Alkylpolyglucoside mit 8 bis 22, vorzugsweise 10 bis 18 Kohlenstoffatomen in der Alkylkette. Diese Verbindungen enthalten meist 1 bis 20, vorzugsweise 1,1 bis 5 Glucosideinheiten.

Eine andere Klasse nichtionischer Tenside sind N-Alkylglucamide der allgemeinen Strukturen

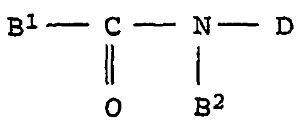

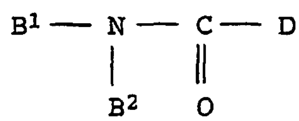

wobei $B_1$ ein $C_6$- bis $C_{22}$-Alkyl, $B^2$ Wasserstoff oder $C_1$- bis $C_4$-Alkyl und D ein Polyhydroxyalkyl-Rest mit 5 bis 12 C-Atomen und mindestens 3 Hydroxygruppen ist. Vorzugsweise steht $B^1$ für $C_{10}$- bis $C_{18}$-Alkyl, $B^2$ für $CH_3$ und D für einen $C_5$- oder $C_6$-Rest. Beispielsweise erhält man derartige Verbindungen durch die Acylierung von reduzierend aminierten Zuckern mit Säurechloriden von $C_{10}$- bis $C_{18}$-Carbonsäuren.

Weitere in Betracht kommende nichtionische Tenside sind die aus der WO-A 95/11225 bekannten endgruppenverschlossenen Fettsäureamidalkoxylate der allgemeinen Formel

$$R^1\text{-CO-NH-}(CH_2)_y\text{-O-}(A^1O)_x\text{-}R^2$$

in der

$R^1$ einen $C_5$- bis $C_{21}$-Alkyl- oder Alkenylrest bezeichnet,

$R^2$ eine $C_1$- bis $C_4$-Alkylgruppe bedeutet,

$A^1$ für $C_2$- bis $C_4$-Alkylen steht,

y die Zahl 2 oder 3 bezeichnet und

x einen Wert von 1 bis 6 hat.

Beispiele für solche Verbindungen sind die Umsetzungsprodukte von n-Butyltriglykolamin der Formel $H_2N-(CH_2-CH_2-O)_3-C_4H_9$ mit Dodecansäuremethylester oder die Reaktionsprodukte von Ethyltetraglykolamin der Formel $H_2N-(CH_2-CH_2-O)_4-C_2H_5$ mit einem handelsüblichen Gemisch von gesättigten $C_8$- bis $C_{18}$-Fettsäuremethylestern.

Weiterhin eignen sich als nichtionische Tenside (D) noch Blockcopolymere aus Ethylenoxid, Propylenoxid und/oder Butylenoxid (Pluronic®- und Tetronic®-Marken der BASF), Polyhydroxy- oder Polyalkoxyfettsäurederivate wie Polyhydroxyfettsäureamide, N-Alkoxy- oder N-Aryloxypolyhydroxyfettsäureamide, Fettsäureamidethoxylate, insbesondere endgruppenverschlossene, sowie Fettsäurealkanolamidalkoxylate.

Die Komponente (D) liegt in der erfindungsgemäßen Textilwaschmittel-Formulierung vorzugsweise in einer Menge von 1 bis 30 Gew.-%, insbesondere 3 bis 25 Gew.-%, vor allem 5 bis 20 Gew.-%, vor.

Man kann einzelne nichtionische Tenside oder eine Kombination unterschiedlicher Niotenside einsetzen. Es können nichtionische Tenside aus nur einer Klasse zum Einsatz gelangen, insbesondere nur alkoxylierte $C_8$- bis $C_{22}$-Alkohole, man kann aber auch Tensidmischungen aus verschiedenen Klassen verwenden.

In einer bevorzugten Ausführungsform enthält die erfindungsgemäße Textilwaschmittel-Formulierung zusätzlich zu den anorganischen Buildern (B) 0,05 bis 20 Gew.-%, insbesondere 1 bis 10 Gew.-% organische Cobuilder in Form von niedermolekularen, oligomeren oder polymeren Carbonsäuren, insbesondere Polycarbonsäuren, oder Phosphonsäuren oder deren Salzen, insbesondere Na- oder K-salzen.

Als organische Cobuilder geeignete niedermolekulare Carbonsäuren oder Phosphonsäuren sind beispielsweise:

Phosphonsäuren wie z.B. 1-Hydroxyethan-1,1-diphosphonsäure, Aminotris(methylenphosphonsäure), Ethylendiamintetra(methylenphosphonsäure), Hexamethylendiamintetra(methylenphosphonsäure) und Diethylentriaminpenta(methylenphosphonsäure);

$C_4$- bis $C_{20}$-Di-, -Tri- und -Tetracarbonsäuren wie z.B. Bernsteinsäure, Propantricarbonsäure, Butantetracarbonsäure, Cyclopentantetracarbonsäure und Alkyl- und Alkenylbernsteinsäuren mit $C_2$- bis $C_{16}$-Alkyl- bzw. -Alkenyl-Resten;

$C_4$- bis $C_{20}$-Hydroxycarbonsäuren wie z.B. Äpfelsäure, Weinsäure, Gluconsäure, Glutarsäure, Citronensäure, Lactobionsäure und Saccharosemono-, di- und tricarbonsäure;

Aminopolycarbonsäuren wie z.B. Nitrilotriessigsäure, β-Alanindiessigsäure, Ethylendiamintetraessigsäure, Serindiessigsäure, Isoserindiessigsäure, Alkylethylendiamintriacetate, N,N-bis(Carboxymethyl)glutaminsäure, Ethylendiamindibernsteinsäure und N-(2-Hydroxyethyl)iminodiessigsäure, Methyl- und Ethylglycindiessigsäure.

Als organische Cobuilder geeignete oligomere oder polymere Carbonsäuren sind beispielsweise:

Oligomaleinsäuren, wie sie beispielsweise in EP-A 451508 und EP-A 396303 beschrieben sind;

Co- und Terpolymere ungesättigter $C_4$-$C_8$-Dicarbonsäuren, wobei als Comonomere monoethylenisch ungesättigte Monomere
aus der Gruppe (i) in Mengen von bis zu 95 Gew.-%,
aus der Gruppe (ii) in Mengen von bis zu 60 Gew.-% und
aus der Gruppe (iii) in Mengen von bis zu 20 Gew.-%
einpolymerisiert sein können.

Als ungesättigte $C_4$-$C_8$-Dicarbonsäuren sind hierbei beispielsweise Maleinsäure, Fumarsäure, Itaconsäure und Citraconsäure geeignet. Bevorzugt wird Maleinsäure.

Die Gruppe (i) umfaßt monoethylenisch ungesättigte $C_3$-$C_8$-Monocarbonsäuren wie z.B. Acrylsäure, Methacrylsäure, Crotonsäure und Vinylessigsäure. Bevorzugt werden aus der Gruppe (i) Acrylsäure und Methacrylsäure eingesetzt.

Die Gruppe (ii) umfaßt monoethylenisch ungesättigte $C_2$-$C_{22}$-Olefine, Vinylalkylether mit $C_1$-$C_8$-Alkylgruppen, Styrol, Vinylester von $C_1$-$C_8$-Carbonsäuren, (Meth)acrylamid und Vinylpyrrolidon. Bevorzugt werden aus der Gruppe (ii) $C_2$-$C_6$-Olefine, Vinylalkylether mit $C_1$-$C_4$-Alkylgruppen, Vinylacetat und Vinylpropionat eingesetzt.

Die Gruppe (iii) umfaßt (Meth)acrylester von $C_1$- bis $C_8$-Alkoholen, (Meth)acrylnitril, (Meth)acrylamide von $C_1$-$C_8$-Aminen, N-Vinylformamid und N-Vinylimidazol.

Falls die Polymeren der Gruppe (ii) Vinylester einpolymerisiert enthalten, können diese auch teilweise oder vollständig zu Vinylalkohol-Struktureinheiten hydrolysiert vorliegen. Geeignete Co-und Terpolymere sind beispielsweise aus US-A 3887806 sowie DE-A 4313909 bekannt.

Als Copolymere von Dicarbonsäuren eignen sich als organische Cobuilder vorzugsweise:

Copolymere von Maleinsäure und Acrylsäure im Gewichtsverhältnis 10:90 bis 95:5, besonders bevorzugt solche im Gewichtsverhältnis 30:70 bis 90:10 mit Molmassen von 1000 bis 150000;

Terpolymere aus Maleinsäure, Acrylsäure und einem Vinylester einer $C_1$-$C_3$-Carbonsäure im Gewichtsverhältnis 10 (Maleinsäure) :90 (Acrylsäure + Vinylester) bis 95 (Maleinsäure) :10 (Acrylsäure + Vinylester), wobei das Gew.-Verhältnis von Acrylsäure zum Vinylester im Bereich von 30:70 bis 70:30 variieren kann;

Copolymere von Maleinsäure mit $C_2$-$C_8$-Olefinen im Molverhältnis 40:60 bis 80:20, wobei Copolymere von Maleinsäure mit Ethylen, Propylen oder Isobuten im Molverhältnis 50:50 besonders bevorzugt sind.

Pfropfpolymere ungesättigter Carbonsäuren auf niedermolekulare Kohlenhydrate oder hydrierte Kohlenhydrate, vgl. US-A 5227446, DE-A 4415623 und DE-A 4313909, eignen sich ebenfalls als organische Cobuilder.

Geeignete ungesättigte Carbonsäuren sind hierbei beispielsweise Maleinsäure, Fumarsäure, Itaconsäure, Citraconsäure, Acrylsäure, Methacrylsäure, Crotonsäure und Vinylessigsäure sowie Mischungen aus Acrylsäure und Maleinsäure, die in Mengen von 40 bis 95 Gew.-%, bezogen auf die zu pfropfende Komponente, aufgepfropft werden.

Zur Modifizierung können zusätzlich bis zu 30 Gew.-%, bezogen auf die zu pfropfende Komponente, weitere monoethylenisch ungesättigte Monomere einpolymerisiert vorliegen. Geeignete modifizierende Monomere sind die oben genannten Monomere der Gruppen (ii) und (iii).

Als Pfropfgrundlage sind abgebaute Polysaccharide wie z.B. saure oder enzymatisch abgebaute Stärken, Inuline oder Zellulose, Eiweißhydrolysate und reduzierte (hydrierte oder hydrierend aminierte) abgebaute Polysaccharide wie z.B. Mannit, Sorbit, Aminosorbit und N-Alkylglucamin geeignet sowie auch Polyalkylenglycole mit Molmassen mit bis zu $M_w$ = 5000 wie z. B. Polyethylenglycole, Ethylenoxid/Propylenoxid- bzw. Ethylenoxid/Butylenoxid- bzw. Ethylenoxid/Propylenoxid/Butylenoxid-Blockcopolymere und alkoxylierte ein- oder mehrwertige $C_1$-$C_{22}$-Alkohole, vgl. US-A 5756456.

Als organische Cobuilder geeignete Polyglyoxylsäuren sind beispielsweise beschrieben in EP-B 001004, US-A 5399286, DE-A 4106355 und EP-A 656914. Die Endgruppen der Polyglyoxylsäuren können unterschiedliche Strukturen aufweisen.

Als organische Cobuilder geeignete Polyamidocarbonsäuren und modifizierte Polyamidocarbonsäuren sind beispielsweise bekannt aus EP-A 454126, EP-B 511037, WO-A 94/01486 und EP-A 581452.

Als organische Cobuilder verwendet man insbesondere auch Polyasparaginsäuren oder Cokondensate der Asparaginsäure mit weiteren Aminosäuren, $C_4$-$C_{25}$-Mono- oder -Dicarbonsäuren und/oder $C_4$-$C_{25}$-Mono- oder -Diaminen. Besonders bevorzugt werden in phosphorhaltigen Säuren hergestellte, mit $C_6$-$C_{22}$-Mono- oder -Dicarbonsäuren bzw. mit $C_6$-$C_{22}$-Mono- oder -Diaminen modifizierte Polyasparaginsäuren eingesetzt.

Als organische Cobuilder eignen sich weiterhin Iminodibernsteinsäure, Oxydibernsteinsäure, Aminopolycarboxylate, Alkylpolyaminocarboxylate, Aminopolyalkylenphosphonate, Polyglutamate, hydrophob modifizierte Citronensäure wie z.B. Agaricinsäure, Poly-$\alpha$-hydroxyacrylsäure, N-Acylethylendiamintriacetate wie Lauroylethylendiamintriacetat und Alkylamide der Ethylendiamintetraessigsäure wie EDTA-Talgamid.

Weiterhin können auch oxidierte Stärken als organische Cobuilder verwendet werden.

In einer weiteren bevorzugten Ausführungsform enthält die erfindungsgemäße Textilwaschmittelformulierung zusätzlich, insbesondere zusätzlich zu den anorganischen Buildern (B), den anionischen Tensiden (C) und/oder den nichtionischen Tensiden (D), 0,5 bis 20 Gew.-%, insbesondere 1 bis 10 Gew.-%, Glycin-N,N-diessigsäure-Derivate, wie sie in der WO 97/19159 beschrieben sind.

In einer weiteren bevorzugten Ausführungsform enthält die erfindungsgemäße Textilwaschmittel-Formulierung zusätzlich 0,5 bis 30 Gew.-%, insbesondere 5 bis 27 Gew.-%, vor allem 10 bis 23 Gew.-% Bleichmittel in Form von Percarbonsäuren, z.B. Diperoxododecandicarbonsäure, Phthalimidopercapronsäure oder Monoperoxophthalsäure oder -terephthalsäure, Addukten von Wasserstoffperoxid an anorganische Salze, z.B. Natriumperborat-Monohydrat, Natriumperborat-Tetrahydrat, Natriumcarbonat-Perhydrat oder Natriumphosphat-Perhydrat, Addukten von Wasserstoffperoxid an organische Verbindungen, z.B. Harnstoff-Perhydrat, oder von anorganischen Peroxosalzen, z.B. Alkalimetallpersulfaten, oder -peroxodisulfaten, gegebenenfalls in Kombination mit 0 bis 15 Gew.-%, vorzugsweise 0,1 bis 15 Gew.-%, insbesondere 0,5 bis 8 Gew.-%, Bleichaktivatoren. Bei Color-Waschmitteln wird das Bleichmittel (wenn vorhanden) in der Regel ohne Bleichaktivator eingesetzt, ansonsten sind üblicherweise Bleichaktivatoren mit vorhanden.

Als Bleichaktivatoren eignen sich:

- polyacylierte Zucker, z.B. Pentaacetylglucose;

- Acyloxybenzolsulfonsäuren und deren Alkali- und Erdalkalimetallsalze, z.B. Natrium-p-nonanoyloxybenzolsulfonat oder Natrium-p-benzoyloxybenzolsulfonat;
- N,N-diacylierte und N,N,N',N'-tetraacylierte Amine, z.B. N,N,N',N'-Tetraacetylmethylendiamin und -ethylendiamin (TAED), N,N-Diacetylanilin, N,N-Diacetyl-p-toluidin oder 1,3-diacylierte Hydantoine wie 1,3-Diacetyl-5,5-dimethylhydantoin;
- N-Alkyl-N-sulfonylcarbonamide, z.B. N-Methyl-N-mesylacetamid oder N-Methyl-N-mesylbenzamid;
- N-acylierte cyclische Hydrazide, acylierte Triazole oder Urazole, z.B. Monoacetylmaleinsäurehydrazid;
- O,N,N-trisubstituierte Hydroxylamine, z.B. O-Benzoyl-N,N-succinylhydroxylamin, O-Acetyl-N,N-succinylhydroxylamin oder O,N,N-Triacetylhydroxylamin;
- N,N'-Diacylsulfurylamide, z.B. N,N'-Dimethyl-N,N'-diacetylsulfurylamid oder N,N'-Diethyl-N,N'-dipropionylsulfurylamid;
- acylierte Lactame wie beispielsweise Acetylcaprolactam, Octanoylcaprolactam, Benzoylcaprolactam oder Carbonylbiscaprolactam;
- Anthranilderivate wie z.B. 2-Methylanthranil oder 2-Phenylanthranil;
- Triacylcyanurate, z.B. Triacetylcyanurat oder Tribenzoylcyanurat;
- Oximester und Bisoximester wie z.B. O-Acetylacetonoxim oder Bisisopropyliminocarbonat;
- Carbonsäureanhydride, z.B. Essigsäureanhydrid, Benzoesäureanhydrid, m-Chlorbenzoesäureanhydrid oder Phthalsäureanhydrid;
- Enolester wie z.B. Isopropenylacetat;
- 1,3-Diacyl-4,5-diacyloxy-imidazoline, z.B. 1,3-Diacetyl-4,5-diacetoxyimidazolin;
- Tetraacetylglycoluril und Tetrapropionylglycoluril;
- diacylierte 2,5-Diketopiperazine, z.B. 1,4-Diacetyl-2,5-diketopiperazin;
- ammoniumsubstituierte Nitrile wie z.B. N-Methylmorpholiniumacetonitrilmethylsulfat;
- Acylierungsprodukte von Propylendiharnstoff und 2,2-Dimethylpropylendiharnstoff, z.B. Tetraacetylpropylendiharnstoff;
- $\alpha$-Acyloxypolyacylmalonamide, z.B. $\alpha$-Acetoxy-N,N'-diacetylmalonamid;
- Diacyl-dioxohexahydro-1,3,5-triazine, z.B. 1,5-Diacetyl-2,4-dioxohexahydro-1.3,5-triazin;
- Benz-(4H)1,3-oxazin-4-one mit Alkylresten, z.B. Methyl, oder aromatischen Resten z.B. Phenyl, in der 2-Position.

Das beschriebene Bleichsystem aus Bleichmitteln und Bleichaktivatoren kann gegebenenfalls noch Bleichkatalysatoren enthalten. Geeignete Bleichkatalysatoren sind beispielsweise quaternierte Imine und Sulfonimine, die beispielsweise beschrieben sind in US-A 5 360 569 und EP-A 453 003. Besonders wirksame Bleichkatalysatoren sind Mangankomplexe, die beispielsweise in der WO-A 94/21777 beschrieben sind. Solche Verbindungen werden im Falle ihres Einsatzes in den Waschmitteln-Formulierungen höchstens in Mengen bis 1,5 Gew.-%, insbesondere bis 0,5 % Gew.-%, im Falle von sehr aktiven Mangankomplexen in Mengen bis zu 0,1 Gew.-%, eingearbeitet.

Neben dem beschriebenen Bleichsystem aus Bleichmitteln, Bleichaktivatoren und gegebenenfalls.Bleichkatalysatoren ist für die erfindungsgemäße Textilwaschmittel-Formulierung auch die Verwendung von Systemen mit enzymatischer Peroxidfreisetzung oder von photoaktivierten Bleichsystemen möglich.

In einer weiteren bevorzugten Ausführungsform enthält die erfindungsgemäße Textilwaschmittel-Formulierung zusätzlich 0,05 bis 4 Gew.-% Enzyme. Vorzugsweise in Waschmitteln eingesetzte Enzyme sind Proteasen, Amylasen, Lipasen und Cellulasen. Von den Enzymen werden vorzugsweise Mengen von 0,1 bis 1,5 Gew.-%, insbesondere vorzugsweise 0,2 bis 1,0 Gew.-%, des konfektionierten Enzyms zugesetzt. Geeignete Proteasen sind z.B. Savinase und Esperase (Hersteller: Novo Nordisk). Eine geeignete Lipase ist z. B. Lipolase (Hersteller: Novo Nordisk). Eine geeignete Cellulase ist z.B. Celluzym (Hersteller: Novo Nordisk). Auch die Verwendung von Peroxidasen zur Aktivierung des Bleichsystems ist möglich. Man kann einzelne Enzyme oder eine Kombination unterschiedlicher Enzyme einsetzen. Gegebenenfalls kann die erfindungsgemäße Textilwaschmittel-Formulierung noch Enzymstabilisatoren, z. B. Calciumpropionat, Natriumformiat oder Borsäuren oder deren Salze, und/oder Oxidationsverhinderer enthalten.

Die erfindungsgemäße Textilwaschmittel-Formulierung kann neben den bisher genannten Hauptkomponenten noch folgende weitere übliche Zusätze in den hierfür üblichen Mengen enthalten:

- kationische Tenside, üblicherweise in einer Menge bis 25 Gew.-%, vorzugsweise 3 bis 15 Gew.-%, beispielsweise $C_8$-bis $C_{16}$-Dialkyldimethylammoniumhalogenide, Dialkoxydimethylammoniumhalogenide oder Imidazoliniumsalze mit langkettigem Alkylrest;

- amphotere Tenside, üblicherweise in einer Menge bis 15 Gew.-%, vorzugsweise 2 bis 10 Gew.-%, beispielsweise Derivate von sekundären oder tertiären Aminen wie z.B. $C_{12}$-$C_{18}$-Alkylbetaine oder $C_{12}$-$C_{18}$-Alkylsulfobetaine oder Aminoxide wie Alkyldimethylaminoxide;

- Vergrauungsinhibitoren und Soil-Release-Polymere (Dabei handelt es sich z.B. um Polyester aus Polyethylenoxiden mit Ethylenglykol und/oder Propylenglykol und aromatischen Dicarbonsäuren oder aromatischen und aliphatischen Dicarbonsäuren oder Polyester aus einseitig endgruppenverschlossenen Polyethylenoxiden mit zwei- und/oder mehrwertigen Alkoholen und Dicarbonsäuren. Derartige Polyester sind bekannt, vgl. beispielsweise US-A-3 557 039, GB-A-1 154 730, EP-A-0 185 427, EP-A-0 241 984, EP-A-0 241 985, EP-A-0 272 033 und US-A-5 142 020. Weitere geeignete Soil-Release-Polymere sind amphiphile Pfropf- oder Copolymere von Vinyl- und/oder Acrylester auf Polyalkylenoxiden, vgl. US-A-4 746 456, US-A-4 846 995, DE-A-3 711 299, US-A-4 904 408, US-A-4 846 994 und US-A-4 849 126, oder modifizierten Cellulosen wie z.B. Methylcellulose, Hydroxylpropylcellulose oder Carboxymethylcellulose. Vergrauungsinhibitoren und Soil-Release-Polymere sind in den Waschmittelformulierungen zu 0,1 bis 2,5 Gew.-%, vorzugsweise zu 0,2 bis 1,5 Gew.-%, besonders bevorzugt zu 0,3 bis 1,2 Gew.-% enthalten. Bevorzugt eingesetzte Soil-Release-Polymere sind die aus der US-A-4 746 456 bekannten Pfropfpolymeren von Vinylacetat auf Polyethylenoxid der Molmasse 2500 - 8000 im Gewichtsverhältnis 1,2:1 bis 3,0:1, sowie handelsübliche Polyethylenterephthalat/Polyoxyethylenterephthalate der Molmasse 3000 bis 25000 aus Polyethylenoxiden der Molmasse 750 bis 5000 mit Terephthalsäure und Ethylenoxid und einem Molverhältnis von Polyethylenterephthalat zu Polyoxyethylenterephthalat von 8:1 bis 1:1 und die aus der DE-A-44 03 866 bekannten Blockpolykondensate, die Blöcke aus (a) Ester-Einheiten aus Polyalkylenglykolen einer Molmasse von 500 bis 7500 und aliphatischen Dicarbonsäuren und/oder Monohydroxymonocarbonsäuren und (b) Ester-Einheiten aus aromatischen Dicarbonsäuren und mehrwertigen Alkoholen enthalten. Diese amphiphilen Blockcopolymerisate haben Molmassen von 1500 bis 25000.);

  - Farbübertragungsinhibitoren, beispielsweise Homo- und Copolymerisate des N-Vinylpyrrolidons, des N-Vinylimidazols, des N-Vinyloxazolidons oder des 4-Vinylpyridin-N-oxids mit Molmassen von 15.000 bis 100.000 sowie vernetzte feinteilige Polymere auf Basis dieser Monomere mit einer Teilchengröße von 0,1 bis 500, vorzugsweise 0,1 bis 250 µm;

  - nichttensidartige Schaumdämpfer oder Schauminhibitoren, beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure;

  - Komplexbildner (auch in der Funktion von organischen Cobuildern);

  - optische Aufheller;

  - Polyethylenglykole;

  - Parfüme oder Duftstoffe;

  - Füllstoffe;

- anorganische Stellmittel, z.B. Natriumsulfat;
- Konfektionierhilfsmittel;
- Löslichkeitsverbesserer;
- Trübungs- und Perlglanzmittel;
- Farbstoffe;
- Korrosionsinhibitoren;
- Peroxidstabilisatoren;
- Elektrolyte.

Eine erfindungsgemäße feste Textilwaschmittel-Formulierung liegt üblicherweise pulver- oder granulatförmig oder in Extrudat- oder Tablettenform vor.

Erfindungsgemäße pulver- oder granulatförmige Waschmittel können bis zu 60 Gew.-% anorganische Stellmittel enthalten. Üblicherweise wird hierfür Natriumsulfat verwendet. Vorzugsweise sind die erfindungsgemäßen Waschmittel aber arm an Stellmitteln und enthalten nur bis zu 20 Gew.-%, besonders bevorzugt nur bis zu 8 Gew.-% an Stellmitteln, insbesondere bei Kompakt- oder Ultrakompaktwaschmitteln. Die erfindungsgemäßen festen Waschmittel können unterschiedliche Schüttdichten im Bereich von 300 bis 1300 g/l, insbesondere von 550 bis 1200 g/l, besitzen. Moderne Kompaktwaschmittel besitzen in der Regel hohe Schüttdichten und zeigen einen Granulataufbau. Zur erwünschten Verdichtung der Waschmittel können die in der Technik üblichen Verfahren eingesetzt werden.

Die erfindungsgemäße Textilwaschmittel-Formulierung wird nach üblichen Methoden hergestellt und gegebenenfalls konfektioniert.

Gegenstand der vorliegenden Erfindung ist auch eine Textilwäschevor- und Textilwäschenachbehandlungs-Formulierung, welche 0,01 bis 40 Gew.-%, insbesondere 0,5 bis 20 Gew.-%, einer erfindungsgemäßen Mischung aus den Komponenten (A) und (B) neben den sonstigen üblichen Bestandteilen enthält. Dabei kann die Zugabe der Komponenten (A) und (B) im Prinzip separat in die Formulierung erfolgen oder es kann die vorgefertigte Mischung aus (A) und (B) in die Formulierung eingearbeitet werden; letztere Methode ist in vielen Fälle die günstigere, insbesondere wenn (A) und (B) im Temperaturbereich von 10 bis 35°C ohne Zusatz von Lösungs- oder Verdünnungsmitteln eine gemeinsame homogene flüssige Phase ausbilden.

Bevorzugt wird hierbei eine Textilwäschevor- und Textilwäschenachbehandlungs-Formulierung, welche weiterhin 1 bis 50 Gew.-%, insbesondere 3 bis 30 Gew.-%, eines oder mehrerer kationischer Tenside aus der Gruppe der quartären Diesterammoniumsalze, der quartären Tetraalkylammoniumsalze, der quartären Diamidoammoniumsalze, der Amidoaminoester und der Imidazoliniumsalze enthält.

Quartäre Diesterammoniumsalze sind insbesondere solche, die zwei $C_{11}$- bis $C_{22}$-Alk(en)ylcarbonyl-oxy-(mono- bis pentamethylen)-Reste und zwei $C_1$- bis $C_3$-Alkyl- oder -Hydroxyalkylreste am quartären N-Atom aufweisen und als Gegenion bei-spielsweise Chlorid, Bromid, Methosulfat oder Sulfat tragen.

Quartäre Diesterammoniumsalze sind weiterhin insbesondere solche, die einen $C_{11}$- bis $C_{22}$-Alk(en)ylcarbonyl-oxy-trimethylen-Rest, der am mittleren C-Atom der Tri-methylen-Gruppierung einen $C_{11}$- bis $C_{22}$-Alk(en)ylcarbonyl-oxy-Rest trägt, und drei $C_1$- bis $C_3$-Alkyl-oder -Hydroxyalkylreste am quartären N-Atom aufweisen und als Gegenion beispielsweise Chlorid, Bromid, Methosulfat oder Sulfat tragen.

Quartäre Tetraalkylammoniumsalze sind insbesondere solche, die zwei $C_1$- bis $C_6$-Alkyl-Reste und zwei $C_8$- bis $C_{24}$-Alk(en)yl-Reste am quartären N-Atom aufweisen und als Gegenion beispielsweise Chlorid, Bromid, Methosulfat oder Sulfat tragen.

Quartäre Diamidoammoniumsalze sind insbesondere solche, die zwei $C_8$- bis $C_{24}$-Alk(en)ylcarbonyl-aminoethylen-Reste, einen Substituenten ausgewählt aus Wasserstoff, Methyl, Ethyl und Polyoxyethylen mit bis zu 5 Oxyethylen-Einheiten und als vierten Reste eine Methylgruppe am quartären N-Atom aufweisen und als Gegenion beispielsweise Chlorid, Bromid, Methosulfat oder Sulfat tragen.

Amidoaminoester sind insbesondere tertiäre Amine, die als Substituenten am N-Atom einen $C_{11}$- bis $C_{22}$-Alk(en)ylcarbonylamino-(mono- bis trimethylen)-Rest, einen $C_{11}$- bis $C_{22}$-Alk(en)ylcarbonyl-oxy-(mono- bis trimethylen)-Rest und eine Methyl-gruppe tragen.

Imidazolinsalze sind insbesondere solche, die in der 2-Position des Heterocyclus einen $C_{14}$- bis $C_{18}$-Alk(en)ylrest, am neutralen N-Atom einen $C_{14}$- bis $C_{18}$-Alk(en)yl-carbonyl-(oxy oder amino)-ethylen-Rest und am die positive Ladung tragenden N-Atom Wasserstoff, Methyl oder Ethyl tragen, Gegenionen sind hierbei beispielsweise Chlorid,

Bromid, Methosulfat oder Sulfat.

Bei den Textilwäschenachbehandlungs-Formulierungen kommen insbesondere Weichspül- und Weichpflegemittel in Betracht.

Sonstige übliche Bestandteile für eine solche Textilwäschevor-und Textilwäsche-nachbehandlungs-Formulierung sind nichtionische Tenside, Duft- und Farbstoffe, Stabilisatoren, Faser- und Farbschutzadditive, Viskositätsmodifizierer, Soil Release Additive, Korrosionsschutzadditive, Bakterizide, Konservierungsmittel und Wasser in den hierfür üblichen Mengen.

Die erfindungsgemäße Mischung aus den Komponenten (A) und (B) eignen sich in hervorragender Weise sowohl, in auf textiles Material aufgebrachter Form, zum Schutz der menschlichen Haut vor schädigender UV-Strahlung als auch zum Schutz von gefärbtem textilem Material vor Verblassung der Farbe. Textiles Material, welches eine derartige erfindungsgemäße Mischung enthält, ist somit ebenfalls Gegenstand der vorliegenden Erfindung.

Mit der erfindungsgemäßen Mischung aus den Komponenten (A) und (B) erzielt man insbesondere höhere UV-Schutzfaktoren (UPF). Weiterhin weist die erfindungsgemäße Mischung eine erhöhte Lichtechtheit aufgrund des verminderten Photoabbaus auf. Weiterhin stellen wäßrige Formulierungen, Zubereitungen und Systeme, die die erfindungsgemäße Mischung enthalten, hinsichtlich der hydrophoben UV-Absorber stabilere Öl-in-Wasser-Emulsionen dar, was auf eine günstigere Teilchengrößenverteilung des hydrophoben UV-Absorber-Systems in der wäßrigen Phase zurückzuführen ist.

Die nachfolgenden Beispiele sollen die vorliegende Erfindung erläutern, ohne sie jedoch zu beschränken.

Beispiel 1 - Anwendung bei der Textilwäschenachbehandlung im Weichspülbad und Bestimmung des UV-Schutzfaktors UPF

Weißes Baumwollgewebe mit einem Flächengewicht von 100g/mm2 und einem UV-Schutz-faktor UPF = 4,50 wurde bei einer Wasserhärte von 3 mmol/l gewaschen. Der Waschprozeß bestand aus einem Hauptwaschgang bei 40°C mit einer handelsüblichen Waschmittelformulierung (Ariel® Color) und anschließendem Weichspülgang. Als Weichspüler wurde eine handelsübliche Formulierung (Lenor® care) in einer Dosierung von 1000 ppm, bezogen auf die Flotte, eingesetzt. Der Weichspüler-Formulierung war entweder kein textilfaseraffiner UV-Absorber oder jeweils 50, 100 oder 200 ppm, bezogen auf die Flotte, einer erfindungsgemäßen UV-Absorber-Mischung M1, M2; M3* oder M4 vor dem Weichspülen zugesetzt worden. Nach dem Weichspülgang wurde das Gewebe entnommen und in getrocknetem Zustand sein UV-Schutzfaktor UPF bestimmt.

Verwendete UV-Absorber-Mischungen M1 bis M4:

M1 = 75 Gew.-% 2-Cyano-3,3-diphenylacrylsäure-2-ethylhexylester (Uvinul® N-539 T) + 25 Gew.-% 2-(4'-Diethylamino-2'-hydroxybenzoyl)-benzoesäure-n-hexylester

M2 = 80 Gew.-% 2-Cyano-3,3-diphenylacrylsäure-2-ethylhexylester + 20 Gew.-% 1-(4-tert.-Butylphenyl)-3-(4-methoxyphenyl)-propan-1,3-dion (Uvinul® BMBM)

M3* = 75 Gew.-% 2-Cyano-3,3-diphenylacrylsäure-2-ethylhexylester + 25 Gew.-% 2-Hydroxy-4-(2-ethylhexyloxy)-benzophenon (Uvi-nul® 408)

M4 = 90 Gew.-% 2-Cyano-3,3-diphenylacrylsäure-2-ethylhexylester + 10 Gew.-% 1,1-Bis-(neopentyloxycarbonyl)-4,4-diphenyl-1,3-butadien

A1 = 100 Gew.-% 2-Cyano-3,3-diphenylacrylsäure-2-ethylhexylester (zum Vergleich)

Tabelle 1 zeigt die Ergebnisse der Versuche:

Tabelle 1

| | UPF mit 50 ppm | UPF mit 100 ppm | UPF mit 200 ppm |
|---|---|---|---|
| Mischung M1 | 10,5 | 15,4 | 24,0 |
| Mischung M2 | 11,1 | 18,2 | 30,9 |

*=nicht erfindungsgemäss

Tabelle 1 (fortgesetzt)

| | UPF mit 50 ppm | UPF mit 100 ppm | UPF mit 200 ppm |
|---|---|---|---|
| Mischung M3 * | 9,6 | 16,4 | 28,2 |
| Mischung M4 | 11,2 | 16,7 | 30,1 |
| | | | |
| UV-Absorber A1 | 8,6 | 13,5 | 21,1 |

* = nicht erfindungsgemä(ss)

Die Ergebnisse belegen, daß der Einsatz der erfindungsgemäßen UV-Absorber-Mischungen M1, M2, M3* oder M4 zu höheren UV-Schutzfaktoren (UPF) des Baumwollgewebes führt als die alleinige Verwendung von A1 in der gleichen Menge.

Beispiel 2 - Anwendung bei der Textilwäschenachbehandlung im Weichspül und Bestimmung der Lichtstabilität des eingesetzten gefärbten Baumwollgewebes

Mit Reaktiv Schwarz 5 gefärbtes Baumwollgewebe (1/3 Richttieftype) wurde im Weichspülgang mit einer handelsüblichen Formulierung (Lenor® care, 1000 ppm bezogen auf die Flotte) unter Zusatz einer erfindungsgemäßen UV-Absorber-Mischung M1, M2, M3, M4, M5, M6 oder M7 (jeweils 200 ppm bezogen auf die Flotte) im Flottenverhältnis 1:12,5 behandelt. Nach dem Weichspülgang wurden die Farbgewebe entnommen und in getrocknetem Zustand auf Lichtstabilität geprüft.

Eingesetzte UV-Absorber-Mischungen M1 bis M7: M1 bis M4: siehe Beispiel 1 (M3 nicht erfindungsgemäss)

M5 = 90 Gew. % 2-Cyano-3,3-diphenylacrylsäure-2-ethylhexylester (Uvinul® N-539 T) + 10 Gew. % 2-(2'-Hydroxy-3',5'-di-tert.-amylphenyl)-2H-benzotriazol (Tinuvin 328)

M6* = 30 Gew. % 2-Cyano-3,3-diphenylacrylsäure-2-ethylhexylester + 70 Gew. % 2-Hydroxy-4-(2-ethylhexyloxy) benzophenon

M7 = 50 Gew. % 2-Cyano-3,3-diphenylacrylsäure-2-ethylhexylester + 50 Gew. % Mischung aus β-[3-(2H-Benzotriazol-2-yl)-5-tert.-butyl-4-hydroxy-phenyl]propionsäure-polyoxyethylenglykolester und Polyoxyethylenglykol-bis-β-[3-(2H-Benzotriazol-2-yl)-5-tert.-butyl-4-hydroxy-phenyl]-propionat mit einem durchschnittlichen Molekulargewicht >600 (Tinuvin® 1130)

A1 = 100 Gew.-% 2-Cyano-3,3-diphenylacrylsäure-2-ethylhexylester (zum Vergleich)

Die Lichtstabilitäten (Photostabilitäten) wurden wie folgt gemessen:

Die Proben wurden in einem Tischbelichtungsgerät SUNTEST® (Fa. Heraeus, Hanau) für 14 Stunden (unter out-door-Bedingungen) belichtet. Von den Färbungen wurden mittels eines Spektralphotometers incl. Integrationskugel ("Lambda 900" der Fa. Perkin Elmer) jeweils die Remissionsspektren vor und nach der Belichtung gemessen. Diese Remissionsspektren wurden entsprechend der Kubelka-Munk-Theorie in K/S-Spektren umgerechnet (K = Absorptionskoeffizient, S = Streukoeffizient). Die Photostabilitäten werden anhand der nach 14-stündiger Belichtung verbleibenden K/S-Werte bezogen auf die K/S-Werte vor Belichtung bewertet (K/S jeweils beim Maximum der K/S-Spektren). Je größer der in Tabelle 2 angegebene Wert in % ist, desto größer ist die Photostabilität.

Tabelle 2 zeigt die Ergebnisse der Messungen:

Tabelle 2

| UV-Absorber | Dosierung [ppm] | K/S (14 h) / K/S (0 h) x 100 % |
|---|---|---|
| ohne | - | 65,2 % |
| A1 | 200 | 67,7 % |
| | | |
| M1 | 200 | 69,7 % |
| M2 | 200 | 70,8 % |
| M3* | 200 | 73,3 % |

*=nicht erfindungsgemäss

Tabelle 2 (fortgesetzt)

| UV-Absorber | Dosierung [ppm] | K/S (14 h) / K/S (0 h) x 100 % |
|---|---|---|
| M4 | 200 | 71,6 % |
| M5 | 200 | 72,9 % |
| M6* | 200 | 76,5 % |
| M7 | 200 | 70,1 % |

*=nicht erfindungsgemäss

Die Ergebnisse belegen, daß der Einsatz der erfindungsgemäßen UV-Absorber-Mischungen M1 bis M7 das Farbgewebe wirksamer vor Farbverblassung durch UV-Strahlen schützt als die alleinige Verwendung von UV-Absorber A1 in der gleichen Menge.

Beispiel 3 - Herstellung von homogenen flüssigen Mischungen aus (A) und (B)

Die nachfolgenden Kombinationen aus Verbindungen (A) und (B) bildeten beim Zusammenmischen bei Raumtemperatur ohne Zusatz von Lösungs- oder Verdünnungsmitteln eine gemeinsame homogene und klare flüssige Phase aus [in Klammern sind beispielhafte Gew.-Mischungsverhältnisse von (A) zu (B) angegeben]. Diese Zusammenstellung ist nur eine exemplarische Auswahl aus den im Rahmen der vorliegenden Erfindung möglichen Mischungen aus (A) und (B), die derartige homogene flüssige Phasen ausbilden.
Als Verbindung (A) wurde in allen Fällen der 2-Ethylhexylester von 2-Cyano-3,3-diphenylacrylsäure (Uvinul® N-539 T) verwendet.
Als Verbindungen (B) wurden eingesetzt : (*-nicht erfindungsgemäss)

2-(2'-Hydroxy-3',5'-di-tert.-amylphenyl)-2H-benzotriazol (Tinuvin® 328) [90:10],
2-(2H-Benzotriazol-2-yl)-4-n-octylphenyl (Tinuvin® 329) [90:10],
2-Hydroxy-3-sec.-butyl-5-tert.-butyl-benzotriazol (Tinuvin® 350) [80:20]*,
Mischung aus β-[3-(2H-Benzotriazol-2-yl)-5-tert.-butyl-4-hydroxyphenyl]-propionsäure-polyoxyethylenglykolester und Polyoxyethylenglykol-bis-β-[3-(2H-Benzotriazol-2-yl)-5-tert.-butyl-4-hydroxyphenyl]-propionat mit einem durchschnittlichen Molekulargewicht >600 (Tinuvin® 1130) [50:50, 20:80],
2-Hydroxy-3-dodecyl-5-methylphenyl-benzotriazol, (Tinuvin® 571) [70:30],
2-Hydroxy-4-methoxy-benzophenon (Uvinul® M40) [80:20]*;
1-(4-tert.-Butylphenyl)-3-(4-methoxyphenyl)-propan-1,3-dion (Uvinul® BMBM) [80:20],
2-Hydroxy-4-(n-octyloxy)-benzophenon (Uvinul® 3008) [75:25]*;
1,1-Bis-(neopentyloxycarbonyl)-4,4-diphenyl-1,3-butadien [90:10],
2-(4'-Diethylamino-2'-hydroxybenzoyl)-benzoesäure-n-hexylester [67:33, 80:20],
2-Hydroxy-4-(2-ethylhexyloxy)-benzophenon [30:70, 75:25]*,
1,1-Dicyano-2-(4-butyloxy)phenyl-2-phenyl-ethen [90:10]*;
1,1-Bis-(neopentyloxycarbonyl)-4,4-diphenyl-1,3-butadien + 2-Hydroxy-4-(2-ethylhexyloxy)-benzophenon [70:10:20],
2-(4'-Diethylamino-2'-hydroxybenzoyl)-benzoesäure-n-hexylester +
2-Hydroxy-4-(2-ethylhexyloxy)-benzophenon [40:10:50],
Mischung aus β-[3-(2H-Benzotriazol-2-yl)-5-tert.-butyl-4-hydroxyphenyl]-propionsäure-polyoxyethylenglykolester und Polyoxyethylenglykol-bis-β-[3-(2H-Benzotriazol-2-yl)-5-tert.-butyl-4-hydroxyphenyl]-propionat mit einem durchschnittlichen Molekulargewicht >600 (Tinuvin® 1130) + 2-Hydroxy-4-(n-octyloxy)-benzophenon (Uvinul® 3008) [45:40:15].

## Patentansprüche

**1.** Verwendung einer Mischung aus

| | | |
|---|---|---|
| (A) | 10 bis 90 Gew.-% | mindestens eines $C_6$- bis $C_{18}$-Alkylesters oder $C_5$- bis $C_8$-Cycloalkylesters der 2-Cyano-3,3-diphenylacrylsäure und |

*=nicht erfindungsgemäss

(fortgesetzt)

| | | |
|---|---|---|
| (B) | 90 bis 10 Gew.-% | mindestens einer weiteren Verbindung, welche mindestens ein UV-Absorptionsmaximum im Bereich von 280 bis 450 nm aufweist und ausgewählt ist aus der Gruppe der |

(a) Phenylbenzotriazole,
(b) Dibenzoylmethane,
(i) Diarylbutadiene und
(j) aminosubstituierten Hydroxybenzophenone,

als textilfaseraffine UV-Absorber.

**2.** Verwendung einer Mischung nach Anspruch 1 aus

(A) 30 bis 70 Gew.-% mindestens einer Verbindung (A) und

(B) 70 bis 30 Gew.-% mindestens einer Verbindung (B).

**3.** Verwendung einer Mischung nach Anspruch 1 oder 2, bei der die Verbindungen (B) mindestens ein UV-Absorptionsmaximum im Bereich von 330 bis 380 nm aufweisen.

**4.** Verwendung einer Mischung nach Anspruch 1 oder 2, bei der die Verbindungen (B) mindestens ein UV-Absorptionsmaximum im Bereich von 280 bis 315 nm (UV-B-Bereich) mit einem $E^1_1$-Wert von mindestens 200 und mindestens ein UV-Absorptionsmaximum im Bereich von 315 bis 400 nm (UV-A-Bereich) mit einem $E^1_1$-Wert von mindestens 200 aufweisen.

**5.** Verwendung einer Mischung nach den Ansprüchen 1 bis 4, bei der die Verbindungen (A) und die Verbindungen (B) im Temperaturbereich von 10 bis 35°C ohne Zusatz von Lösungs- oder Verdünnungsmitteln eine gemeinsame homogene flüssige Phase ausbilden.

**6.** Verwendung einer Mischung nach den Ansprüchen 1 bis 5, bei der die Verbindungen (B) einen n-Octanol/Wasser-Verteilungskoeffizienten log P von mindestens 1,9 aufweisen.

**7.** Verwendung einer Mischung nach den Ansprüchen 1 bis 6 als textilfaseraffine UV-Absorber für textiles Material, das aus Cellulose besteht oder Cellulose enthält.

**8.** Verfahren zum Schutz der menschlichen Haut vor schädigender UV-Strahlung, **dadurch gekennzeichnet, daß** man eine Mischung gemäß den Ansprüchen 1 bis 6 auf textiles Material bei der Textilausrüstung, der Textilwäsche und/oder der Textilwäschevor- oder Textilwäschenachbehandlung aufbringt.

**9.** Verfahren zum Schutz von gefärbtem textilem Material vor Verblassung der Farbe, **dadurch gekennzeichnet, daß** man eine Mischung gemäß den Ansprüchen 1 bis 6 auf textiles Material bei der Textilausrüstung, der Textilwäsche und/oder der Textilwäschevor- oder Textilwäschenachbehandlung aufbringt.

**10.** Textilwaschmittel-Formulierung, enthaltend 0,01 bis 20 Gew.-% einer Mischung gemäß den Ansprüchen 1 bis 6 neben den sonstigen üblichen Bestandteilen.

**11.** Textilwäschevor- und Textilwäschenachbehandlungs-Formulierung, enthaltend 0,01 bis 40 Gew.-% einer Mischung gemäß den Ansprüchen 1 bis 6 neben den sonstigen üblichen Bestandteilen.

**12.** Textilwäschevor- und Textilwäschenachbehandlungs-Formulierung nach Anspruch 11, enthaltend weiterhin 1 bis 50 Gew.-% eines oder mehrerer kationischer Tenside aus der Gruppe der quartären Diesterammoniumsalze, der quartären Tetraalkylammoniumsalze, der quartären Diamidoammoniumsalze, der Amidoaminoester und der Imidazoline.

**13.** Textiles Material, enthaltend eine Mischung gemäß den Ansprüchen 1 bis 6.

**14.** Mischung aus

| | | |
|---|---|---|
| (A) | 10 bis 90 Gew.-% | mindestens eines $C_6$- bis $C_{18}$-Alkylesters oder $C_5$- bis $C_8$-Cycloalkylesters der 2-Cyano-,3-diphenylacrylsäure und |
| (B) | 90 bis 10 Gew.-% | mindestens einer weiteren Verbindung, welche mindestens ein UV-Absorptionsmaximum im Bereich von 280 bis 450 nm aufweist und ausgewählt ist aus der Gruppe der |

(a) Phenylbenzotriazole,
(i) Diarylbutadiene und
(j) aminosubstituierten Hydroxybenzophenone.

**15.** Verwendung von UV-Absorbern aus der Gruppe der Diarylbutadiene und der aminosubstituierten Hydroxybenzophenone als textilfaseraffine UV-Absorber in auf textiles Material aufgebrachter Form zum Schutz der menschlichen Haut vor schädigender UV-Strahlung und/oder zum Schutz von gefärbtem textilem Material vor Verblassung der Farbe.

## Claims

**1.** The use of a mixture comprising

| | | |
|---|---|---|
| (A) | from 10 to 90% | by weight of at least one $C_6$- to $C_{18}$-alkyl ester or $C_5$- to $C_8$-cycloalkyl ester of 2-cyano-3,3-diphenylacrylic acid and |
| (B) | from 90 to 10% | by weight of at least one further compound which has at least one UV absorption maximum in the range from 280 to 450 nm and is selected from the group consisting of |

(a) phenylbenzotriazoles,
(b) dibenzoylmethanes,
(i) diarylbutadienes and
(j) amino-substituted hydroxybenzophenones,

as UV absorbers possessing affinity for textile fiber.

**2.** The use as claimed in claim 1 of a mixture comprising

(A) from 30 to 70% by weight of at least one compound (A) and

(B) from 70 to 30% by weight of at least one compound (B).

**3.** The use as claimed in claim 1 or 2 of a mixture wherein said compounds (B) have at least one UV absorption maximum in the range from 330 to 380 nm.

**4.** The use as claimed in claim 1 or 2 of a mixture wherein said compounds (B) have at least one UV absorption maximum in the range from 280 to 315 nm (UV-B range) with an $E^1_1$ value of at least 200 and at least one UV absorption maximum in the range from 315 to 400 nm (UV-A range) with an $E^1_1$ value of at least 200.

**5.** The use as claimed in any of claims 1 to 4 of a mixture wherein said compounds (A) and said compounds (B) form a common homogeneous liquid phase in the temperature range from 10 to 35°C without solvents or diluents being added.

**6.** The use as claimed in any of claims 1 to 5 of a mixture wherein said compounds (B) have an n-octanol/water partition coefficient log P of at least 1.9.

**7.** The use as claimed in any of claims 1 to 6 of a mixture as UV absorbers for cellulosic textile material that possess affinity for textile fiber.

**8.** A method of protecting human skin against harmful UV radiation, which comprises applying a mixture as set forth in any of claims 1 to 6 to textile material in the course of textile finishing, laundering and/or laundry pre- or aftertreatment.

**9.** A method of protecting dyed textile material against fading, which comprises applying a mixture as set forth in any of claims 1 to 6 to textile material in the course of textile finishing, laundering and/or laundry pre- or aftertreatment.

**10.** A laundry detergent comprising from 0.01 to 20% by weight of a mixture as set forth in any of claims 1 to 6 as well as other, customary ingredients.

**11.** A laundry pre- or aftertreatment comprising from 0.01 to 40% by weight of a mixture as set forth in any of claims 1 to 6 as well as other, customary ingredients.

**12.** A laundry pre- or aftertreatment as claimed in claim 11, further comprising from 1 to 50% by weight of one or more cationic surfactants selected from the group consisting of quaternary diesterammonium salts, quaternary tetraalkylammonium salts, quaternary diamidoammonium salts, amidoamino esters and imidazolines.

**13.** A textile material comprising a mixture as set forth in any of claims 1 to 6.

**14.** A mixture comprising

| | | |
|---|---|---|
| (A) | from 10 to 90% | by weight of at least one $C_6$- to $C_{18}$-alkyl ester or $C_5$- to $C_8$-cycloalkyl ester of 2-cyano-3,3-diphenylacrylic acid and |
| (B) | from 90 to 10% | by weight of at least one further compound which has at least one UV absorption maximum in the range from 280 to 450 nm and is selected from the group consisting of |

(a) phenylbenzotriazoles,
(i) diarylbutadienes and
(j) amino-substituted hydroxybenzophenones.

**15.** The use of UV absorbers from the group consisting of diarylbutadienes and amino-substituted hydroxybenzophenones as UV absorbers possessing affinity for textile fiber on textile material to protect human skin against harmful UV radiation and/or to protect dyed textile material against fading.

**Revendications**

**1.** Utilisation d'un mélange

(A) de 10 à 90 % en poids d'au moins un ester alkylique en $C_6$-$C_{18}$ ou d'un ester cycloalkylique en $C_5$-$C_8$ de l'acide 2-cyano-3,3-diphénylacrylique, et
(B) de 90 à 10 % en poids d'au moins un autre composé qui présente au moins un maximum d'absorption des U.V. dans la gamme de 280 à 450 nm et qui est choisi parmi le groupe

(a) des phénylbenzotriazoles,
(b) des dibenzoylméthanes,
(i) des diarylbutadiènes, et
(j) des hydroxybenzophénones aminosubstituées, comme agent absorbant les U.V. à affinité pour les fibres textiles.

**2.** Utilisation d'un mélange suivant la revendication 1, à base

(A) de 30 à 70 % en poids d'au moins un composé (A), et
(B) de 70 à 30 % en poids d'au moins un composé (B).

**3.** Utilisation d'un mélange suivant la revendication 1 ou 2, dans lequel les composés (B) présentent au moins un maximum d'absorption des U.V. dans la gamme de 330 à 380 nm.

4. Utilisation ;d'un mélange suivant la revendication 1 ou 2, dans lequel les composés (B) présentent au moins un maximum d'absorption des U.V. dans la gamme de 280 à 315 nm (gamme des U.V.-B) avec une valeur $E^1_1$ d'au moins 200 et au moins un maximum d'absorption des U.V. dans la gamme de 315 à 400 nm (gamme des U.V.-A) avec une valeur $E^1_1$ d'au moins 200.

5. Utilisation d'un mélange suivant les revendications 1 à 4, dans lequel les composés (A) et les composés (B) réalisent dans la gamme de températures de 10 à 35°C une phase liquide homogène commune, sans addition de solvants ou de diluants.

6. Utilisation d'un mélange suivant les revendications 1 à 5, dans lequel les composés (B) présentent un coefficient de distribution de n-octanol/eau log P d'au moins 1,9.

7. Utilisation d'un mélange suivant les revendications 1 à 6, comme agent d'absorption des U.V. à affinité pour les fibres textiles, pour une matière textile qui est constituée de cellulose ou qui contient de la cellulose.

8. Procédé de protection de la peau humaine vis-à-vis d'un rayonnement U.V. nuisible, **caractérisé en ce qu'**on applique un mélange suivant les revendications 1 à 6 sur une matière textile lors de l'apprêt des textiles, du lavage des textiles et/ou du traitement avant ou après le lavage des textiles.

9. Procédé de protection de matière textile colorée avant que sa couleur ne se perde, **caractérisé en ce qu'**on applique un mélange suivant les revendications 1 à 6 sur une matière textile lors de l'apprêt des textiles, du lavage des textiles et/ou du traitement avant ou après le lavage des textiles.

10. Formulation de produit de lavage de textile, contenant 0,01 à 20 % en poids d'un mélange suivant les revendications 1 à 6, outre les autres composants courants.

11. Formulation pour le traitement avant et après le lavage des textiles, contenant 0,01 à 40 % en poids d'un mélange suivant les revendications 1 à 6, outre les autres composants courants.

12. Formulation pour le traitement avant et après le lavage des textiles suivant la revendication 11, contenant par ailleurs 1 à 50 % en poids d'un ou de plusieurs agents tensioactifs cationiques du groupe des sels de diesterammonium quaternaire, des sels de tétraalkyl-ammonium quaternaire, des sels de diamido-ammonium quaternaire, des amidoaminoesters et des imidazolines.

13. Matière textile contenant un mélange suivant les revendications 1 à 6.

14. Mélange à base

(A) de 10 à 90 % en poids d'au moins un ester alkylique en $C_6$-$C_{18}$ ou d'un ester cycloalkylique en $C_5$-$C_8$ de l'acide 2-cyano-3,3-diphénylacrylique, et
(B) de 90 à 10 % en poids d'au moins un autre composé qui présente au moins un maximum d'absorption des U.V. dans la gamme de 280 à 450 nm et qui est choisi parmi le groupe

(a) des phénylbenzotriazoles,
(i) des diarylbutadiènes, et
(j) des hydroxybenzophénones aminosubstituées.

15. Utilisation d'agents absorbant les U.V. du groupe des diarylbutadiènes et des hydroxybenzophénones aminosubstituées, comme agents d'absorption des U.V. à affinité pour les fibres textiles, sous une forme appliquée sur une matière textile, pour protéger la peau humaine du rayonnement U.V. nuisible et/ou pour protéger la matière textile colorée d'une perte de la couleur.